# EUROPEAN PATENT APPLICATION

(11) **EP 4 646 992 A1**
(43) Date of publication of application: **12.11.2025**
(21) Application number: 25174371.2
(22) Date of filing: 06.05.2025
(51) Int. Cl.: A61B 1/00, A61B 1/24

(54) **INTRAORAL IMAGING APPARATUS AND INTRAORAL IMAGE DISPLAYING METHOD**

(30) Priority: 07.05.2024 KR 20240059760; 21.04.2025 KR 20250051811
(71) Applicant: Rayence Co., Ltd., Hwaseong-si, Gyeonggi-do 18449 (KR); Vatech Ewoo Holdings Co., Ltd., Hwaseong-si, Gyeonggi-do, 18449 (KR)
(72) Inventor: Kim, Tae Woo, 18449 Hwaseong-si (KR); Park, Seok Woo, 18449 Hwaseong-si (KR)
(74) Representative: Lorenz & Kollegen

(57) **Abstract**

Proposed is an intraoral image displaying method. The intraoral image displaying method may include storing a plurality of optical images for the intraoral cavity including a teeth arrangement, generating an optical panoramic image for the entire teeth arrangement by stitching the plurality of optical images along a teeth arrangement direction, and displaying the optical panoramic image.

## Description

### CROSS REFERENCE TO RELATED APPLICATION

The present application claims priority to Korean Patent Application No. 10-2024-0059760, filed May 07, 2024, No. 10-2025-0051811, filed April 21, 2025, the entire contents of which are incorporated herein for all purposes by this reference.

### BACKGROUND

### Technical Field

The present disclosure relates to an intraoral imaging apparatus and an intraoral imaging method.

### Description of the Related Art

An optical-based intraoral imaging apparatus generally includes an intraoral camera. The optical-based intraoral imaging apparatus may be configured to acquire an image of a state and a lesion of a tooth by irradiating the tooth with light from a light source and by converting light reflected and transmitted from the tooth into an electrical signal.

As an example, the intraoral camera may include a color mode in which a color image is acquired by using white light, a transillumination mode in which a transillumination image is acquired by using red light, and a fluorescence mode in which a fluorescence image is acquired by using blue light.

However, the intraoral camera has a limited FOV (Field of View), so that the intraoral camera is capable of displaying only about one to three teeth in a single individual image. Accordingly, when the intraoral camera is used, it is difficult to identify a position and a type of each of tooth within individual images, and it is difficult to identify a relationship between the individual images, so that the intraoral camera is not suitable for acquiring and viewing the overall state of the teeth.

A technology which becomes a background of the present disclosure is disclosed in Korean Patent No. 10-1686306.

### SUMMARY

Accordingly, the present disclosure has been made keeping in mind the above problems occurring in the related art, and an objective of the present disclosure is to provide an intraoral imaging apparatus and an intraoral image displaying method, the apparatus and the method being capable of generating and providing an optical panoramic image for the entire teeth arrangement by stitching a plurality of individual images along a teeth arrangement direction, thereby supporting a comprehensive observation of information for a lesion of a tooth and a comprehensive identification of a relationship between the tooth where the lesion is present and a state of surrounding teeth.

However, the problems to be solved by the present disclosure are not limited to the above-described problems, and there may be other problems to be solved by the present disclosure.

As a technical mechanism for realizing the technical objective of the present disclosure, according to an aspect of the present disclosure, there is provided an intraoral image displaying method including: storing a plurality of optical images for the intraoral cavity including a teeth arrangement; generating an optical panoramic image for the entire teeth arrangement by stitching the plurality of optical images along a teeth arrangement direction; and displaying the optical panoramic image.

As a technical mechanism for realizing the technical objective of the present disclosure, according to an aspect of the present disclosure, there is provided an intraoral imaging apparatus including: an optical image storage part in which a plurality of optical images for the intraoral cavity including a teeth arrangement is stored; an image generation part configured to generate an optical panoramic image for the entire teeth arrangement by stitching the plurality of optical images along a teeth arrangement direction; and a display part configured to display the optical panoramic image.

As a technical mechanism for realizing the technical objective of the present disclosure, according to an aspect of the present disclosure, there is provided an intraoral imaging system including: an intraoral photographing apparatus configured to photograph a plurality of optical images for the intraoral cavity including a teeth arrangement; and an intraoral imaging apparatus configured to generate an optical panoramic image for the entire teeth arrangement by stitching the plurality of optical images for the intraoral cavity along a teeth arrangement direction, the intraoral imaging apparatus being configured to display the optical panoramic image.

The above-described technical solutions are set forth to illustrate only and should not be construed as intended to limit the present disclosure. In addition to the exemplary embodiments described above, additional embodiments may exist in the drawings and detailed description of the present disclosure.

According to an aspect of the present disclosure, since the optical panoramic image for the entire teeth arrangement is generated and provided by stitching the plurality of individual images along the teeth arrangement direction, the present disclosure may support a comprehensive observation of a dental lesion and a comprehensive observation of a relationship between the tooth where the lesion is present and a state of surrounding teeth.

However, the effects capable of being acquired from the present disclosure are not limited to the effects described above, and other effects may also exist.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a view schematically illustrating a configuration of an intraoral imaging system according to an embodiment of the present disclosure;
FIG. 2A to FIG. 2C are views illustrating types of optical images according to an embodiment of the present disclosure;
FIG. 3A to FIG. 3D are views illustrating a process of generating an optical panoramic image on the basis of a plurality of optical images according to an embodiment of the present disclosure;
FIG. 4A and FIG. 4B are views illustrating an example of an X-ray panoramic image and an optical panoramic image according to an embodiment of the present disclosure;
FIG. 5 is a block diagram schematically illustrating an intraoral imaging apparatus according to an embodiment of the present disclosure; and
FIG. 6 is an operation flow chart illustrating an intraoral image displaying method according to an embodiment of the present disclosure.

### DETAILED DESCRIPTION

Hereinafter, embodiments of the present disclosure will be described in detail such that those skilled in the art to which the present disclosure belongs may easily implement the present disclosure with reference to the accompanying drawings. However, the present disclosure may be implemented in many different forms and is not limited to the embodiments to be described herein. In addition, in order to clearly describe the present disclosure with reference to the drawings, parts irrelevant to the description are omitted, and similar reference numerals denote similar parts throughout the specification.

Throughout the present specification, when a part is described to be "connected" to another part, this includes not only a case where the part is "directly connected" thereto, but also a case where the part is "indirectly connected" or "electrically connected" thereto with another element therebetween.

Throughout the present specification, when a member is referred to as being "on", "on an upper portion of", "on an upper end of", "under", "on a lower portion of", or "on a lower end of" another member, this may include not only the case where a member is in contact with another member, but also the case where another member exists between two members.

Throughout the present specification, when a part is referred to as "including" an element, it means that the part may include other elements as well without excluding the other elements unless specifically stated otherwise.

The present disclosure relates to an intraoral apparatus and an intraoral image displaying method.

Throughout the present specification, the term "subject" may refer to a person who will receive a diagnosis and an observation of teeth (intraoral cavity) using a dental image displaying system according to an embodiment of the present disclosure, i.e., a patient. Furthermore, the term "user" may refer to a person performing the diagnosis and the observation of the subject's teeth (intraoral cavity) by using the dental image displaying system according to an embodiment of the present disclosure, i.e., a medical personnel member (a doctor, a nurse, and so on). However, the meaning of the terms are not limited thereto.

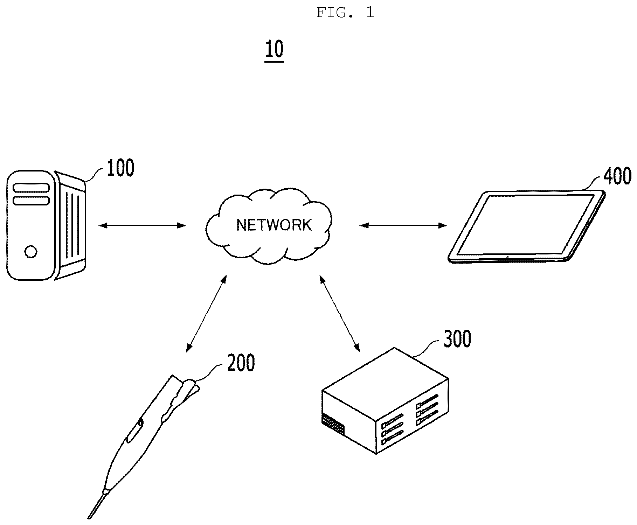

FIG. 1 is a view schematically illustrating an intraoral image displaying system according to an embodiment of the present disclosure.

Referring to FIG. 1, an intraoral imaging system 10 may include an intraoral imaging apparatus 100 (hereinafter, referred to as an "imaging apparatus 100"), an intraoral photographing apparatus 200, an X-ray radiography apparatus 300, and a display device 400.

According to an embodiment of the present disclosure, the imaging apparatus 100 may be intended to display an intraoral image acquired from the intraoral photographing apparatus 200 and the X-ray radiography apparatus 300 through the display device 400. Specifically, on the basis of a plurality of optical images 101 acquired for about one to three teeth, the imaging apparatus 100 may generate and display an optical panoramic image 102 for the entire teeth arrangement, such as an X-ray panoramic image 201 radiographed by the X-ray radiography apparatus 300.

At this time, the intraoral photographing apparatus 200 may refer to an optical-based intraoral photographing apparatus such as an intraoral camera. Specifically, the intraoral photographing apparatus 200 may be configured to photograph a tooth on the basis of reflected light or transmitted light detected from the tooth by irradiating the tooth with light in a specific wavelength range. An image of a specific tooth among the plurality of teeth is capable of being acquired by using the intraoral photographing apparatus 200, but there is a disadvantage that an image of the entire tooth is difficult to be displayed by using the intraoral photographing apparatus 200.

In addition, the X-ray radiography apparatus 300 is configured to provide an X-ray image by radiographing an X-ray image on an intraoral and maxillofacial region of the subject. For example, the X-ray radiography apparatus 300 may be a panoramic X-ray radiography apparatus configured to display the overall projected structure of the intraoral and maxillofacial region in a planar format. The X-ray radiography apparatus 300 has an advantage that the entire teeth arrangement is capable of being observed by using the X-ray radiography apparatus 300, but has a disadvantage that the X-ray radiography apparatus 300 is not suitable for identifying a lesion present in individual teeth and is not suitable for diagnosing the lesion.

Accordingly, the imaging apparatus 100 may display the optical panoramic image 102 generated on the basis of the plurality of optical images 101 taken by the intraoral photographing apparatus 200 in conjunction with the X-ray panoramic image 201 taken by the X-ray radiography apparatus 300 on the display device 400.

Here, the display device 400 may be configured to output the plurality of optical images 101, the optical panoramic image 102, and the X-ray panoramic image 201, and so on displayed on the display device 400. For example, the display device 400 may refer to at least one selected from a smartphone, a smart pad, a tablet PC, a wearable device, all kinds of wireless communication devices such as a Personal Communication System (PCS), a Global System for Mobile communication (GSM), a Personal Digital Cellular (PDC), a Personal Handyphone System (PHS), a Personal Digital Assistant (PDA), an International Mobile Telecommunication (IMT)-2000, a Code Division Multiple Access (CDMA)-2000, a Wideband Code Division Multiple Access (W-CDMA), a Wireless Broadband Internet (WIBRO) terminal, a fixed-type terminal such as a desktop computer and a smart TV, and various types of display devices capable of displaying an image, such as an LCD display, an LED display, an AMOLED display, a CRT display, and so on, but is not limited thereto.

In addition, the imaging apparatus 100, the intraoral photographing apparatus 200, the X-ray radiography apparatus 300, and the display device 400 may be linked with each other through a network and may transmit and receive data. At this time, for example, the network may include a 3rd Generation Partnership Project (3GPP) network, a Long Term Evolution (LTE) network, a 5G network, a World Interoperability for Microwave Access (WIMAX) network, a wired/wireless Internet, a Local Area Network (LAN), a Wireless Local Area Network (Wireless LAN), a Wide Area Network (WAN), a Personal Area Network (PAN), a Bluetooth network, a Wi-Fi network, a Near Field Communication (NFC) network, a satellite broadcasting network, an analog broadcasting network, a Digital Multimedia Broadcasting (DMB) network, and so on, but is not limited thereto.

In addition, the imaging apparatus 100 may further include an input part, a controller, and a storage part.

The input part may be configured to receive a user input. For example, the input part may include a mouse, a keyboard of a computer, a keypad, a touchpad, and so on, but is not limited thereto. Furthermore, the input part may include a Graphic User Interface (GUI) that can be controlled with the input part.

The controller may refer to a central processing unit configured to control an overall operation of the imaging apparatus 100. For example, the controller may be implemented by using at least one selected from Application Specific Integrated Circuits (ASICs), Digital Signal Processors (DSPs), Digital Signal Processing Devices (DSPDs), Programmable Logic Devices (PLDs), Field-Programmable Gate Arrays (FPGAs), processors, controllers, micro-controllers, and microprocessors, or may be implemented as a firmware module or a software module capable of being executed on a hardware platform described above. At this time, the firmware module or the software module may be implemented by one or more software applications written in an appropriate program language.

The storage part may be configured to store various images, a variable value, initial projection data, the plurality of optical images 101, the optical panoramic image 102, the X-ray panoramic image 201, and so on for controlling the operation of the imaging apparatus 100. For example, the storage part may be implemented as one storage medium of a flash memory type, a hard disk type, a MultiMedia Card (MMC) type, a card type memory (for example, a Secure Digital (SD) card or an eXtream Digital (XD) card), a Random Access Memory (RAM), a Static Random Access Memory (SRAM), a Read-Only Memory (ROM), an Electrically Erasable Programmable Read-Only Memory (EEPROM), a Programmable Read-Only Memory (PROM), a magnetic memory, a magnetic disk, and an optical disk, but is not limited thereto.

Hereinafter, a process of generating and displaying the optical panoramic image 102 according to an embodiment of the present disclosure will be described in detail.

According to an embodiment of the present disclosure, the imaging apparatus 100 may acquire the plurality of optical images 101 taken from the intraoral photographing apparatus 200. At this time, the plurality of optical images 101 may be an image in which at least one side surface of the occlusion surface, the lingual side, and the labial side of the teeth of the subject person.

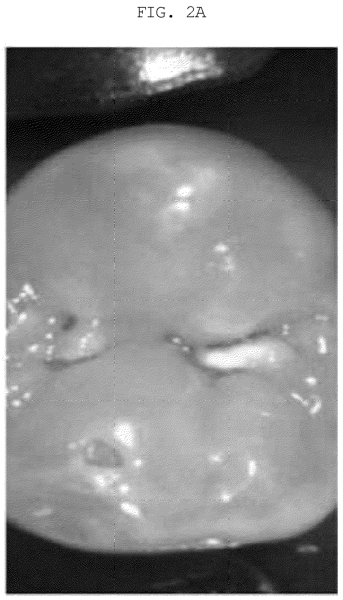

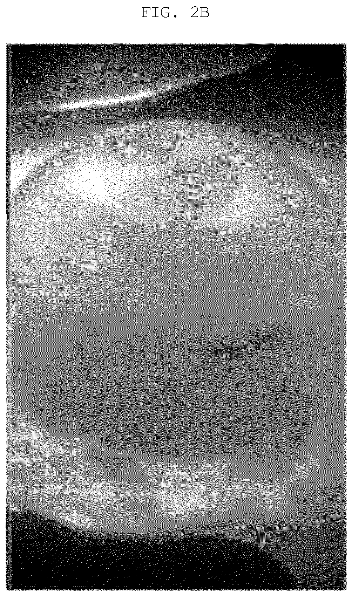

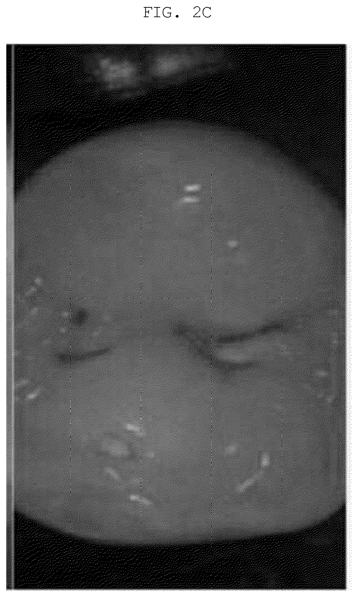

FIG. 2A to FIG. 2C are views illustrating types of optical images according to an embodiment of the present disclosure. Specifically, FIG. 2A to FIG. 2C may illustrate a color image (see FIG. 2A), a transillumination image (see FIG. 2B), and a fluorescence image (see FIG. 2C) that can be acquired from the intraoral photographing apparatus 200.

In this regard, the intraoral photographing apparatus 200 may include a white light source emitting light in a visible light wavelength range of about 400 nm to 700 nm, may detect a reflected light emitted from the white light source toward a tooth, and then may generate a color image. At this time, the color image may be a detailed display of the appearance of the tooth.

In addition, the intraoral photographing apparatus 200 may include a red light source emitting light in an infrared wavelength range of about 850 nm to 950 nm, may detect a reflected light emitted from the red light source toward a tooth and/or a transmitted light through the tooth, and then may generate a reflection illumination image and/or a transillumination image. At this time, as illustrated in FIG. 2B, the reflection illumination image and/or the transillumination image using the red light source may display lesions such as a tooth crack and a tooth caries more darkly so that the lesions are capable of being identified.

In addition, the intraoral photographing apparatus 200 may include a blue light source emitting light in an ultraviolet wavelength range of about 385 nm to 405 nm, may detect a reflected light emitted from the blue light source toward a tooth, and then may generate a fluorescence image. At this time, the fluorescence image may display lesions such as a tooth plaque, a tooth caries, and so on in red so that the lesions are capable of being identified.

In addition, the intraoral photographing apparatus 200 may be configured to spectrally resolve the reflected light and/or the transmitted light of visible light, infrared light, and ultraviolet light into a plurality of spectral bands (including hyperspectral), and may generate at least one spectral band image from the resolved light.

Accordingly, the plurality of optical images described below may include at least one image selected from the color image, the transillumination image, and the fluorescence image described above. That is, the plurality of optical images may be images acquired by using at least one light source selected from the white light source, the red light source, and the blue light source.

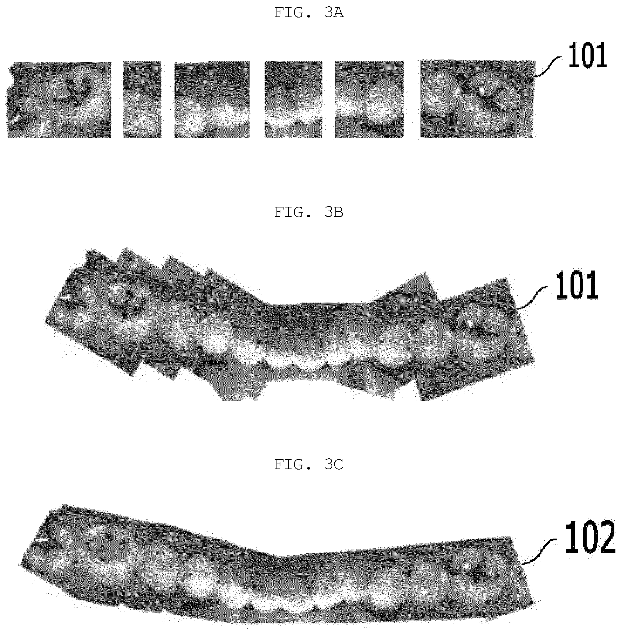

FIG. 3A to FIG. 3D are views illustrating a process of generating an optical panoramic image on the basis of a plurality of optical images according to an embodiment of the present disclosure.

FIG. 3A may be a view exemplarily illustrating the plurality of optical images 101 acquired (taken) by the intraoral photographing apparatus 200. In this regard, as illustrated in FIG. 3A, the intraoral photographing apparatus 200 may be configured to acquire an individual optical image taken such that about one to three teeth are continuously acquired according to a teeth direction of the subject person. In addition, the plurality of optical images 101 of the intraoral cavity including a teeth arrangement photographed by the intraoral photographing apparatus 200 may be stored in the imaging apparatus 100.

Referring to FIG. 3B and FIG. 3C, the imaging apparatus 100 may generate the optical panoramic image 102 for the entire teeth arrangement by stitching the acquired plurality of optical images 101 along a direction of the teeth arrangement. Specifically, the imaging apparatus 100 may be configured to continuously place each of the plurality of optical images 101 such that each of the plurality of optical images 101 corresponds to the teeth arrangement of the subject person (see FIG. 3B), and may be configured to stitch each of the placed optical images 101 to each other and to reconstruct each of the placed optical images 101 into a single image (see FIG. 3C).

At this time, the imaging apparatus 100 may generate the optical panoramic image 102 on the basis of a tooth number for at least one tooth included in each of the plurality of optical images 101. The tooth number is assigned to identify each tooth according to the type and position of the plurality of teeth, and may be assigned on the basis of a sensor value acquired from an inertial sensor included in the intraoral photographing apparatus 200 configured to photograph the plurality of optical images 101, the sensor value being acquired when the plurality of optical images 101 is acquired.

That is, the intraoral photographing apparatus 200 may include the inertial sensor. The inertial sensor may exemplarily be a three-axis sensor, and may be configured to measure a sensor value for an angle of the intraoral photographing apparatus 200 that is tilted according to the type and position of the teeth when a medical personnel member photographs the optical images 101 of the teeth of the subject person by using the intraoral photographing apparatus 200.

By using the inertial sensor, the imaging apparatus 100 may assign a unique tooth number for the sequentially acquired corresponding optical images 101 on the basis of a measurement value of the inertial sensor measured when the corresponding optical images 101 are acquired. As the optical images 101 display only one to three teeth, the imaging apparatus 100 may utilize the tooth number so as to prevent a situation in which an order of each of the optical images 101 is changed differently from the teeth arrangement of the subject person or some optical images 101 are omitted when the stitching of the plurality of optical images 101 is performed.

However, the present disclosure is not limited thereto. Furthermore, the tooth number may be assigned according to an order in which the teeth are photographed when the medical personnel sequentially acquires the plurality of optical images 101 according to a predetermined order, or may be assigned through a learning model trained by inputting optical images so as to identify the position and type of the teeth included in the corresponding optical images. Such a method of assigning the tooth number may be set and changed according to the user input.

In other words, the imaging apparatus 100 may be disposed such that the plurality of optical images 101 is corresponding to the teeth of the subject person according to a predetermined tooth number. Here, the imaging apparatus 100 does not simply list the plurality of optical images 101 according to the tooth number, but may be configured to place each of the optical images 101 such that the angle of each of the optical images is adjusted so that the size and shape of the teeth overlapped and included in the consecutive optical images 101 are not distorted by considering the size and shape of at least one tooth that is included in each of the plurality of optical images 101, and then may generate one optical panoramic image 102 by stitching between each of the optical images 101.

At this time, the imaging apparatus 100 may generate the optical panoramic image 102 in a linear shape or an arch shape. As an example, the imaging apparatus 100 may determine a shape of the optical panoramic image 102 according to a side from which the plurality of optical images 101 is taken.

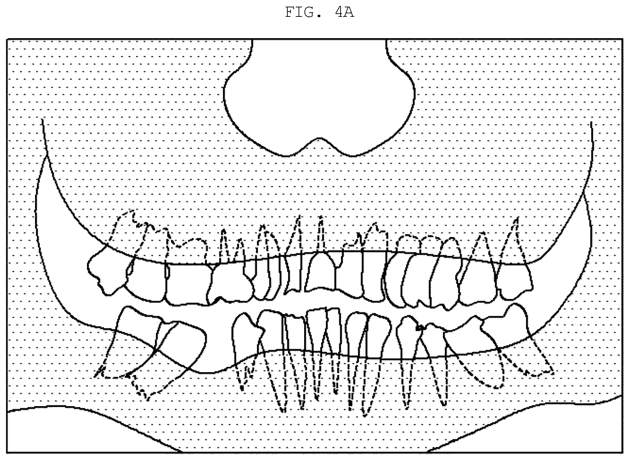

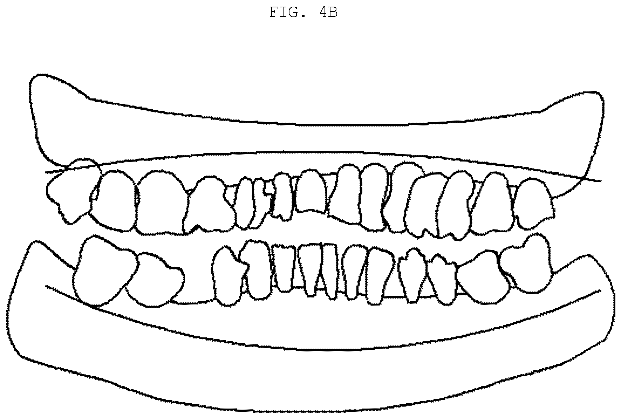

Specifically, when the plurality of optical images 101 is a plurality of images taken by photographing the occlusion surface of the teeth, the imaging apparatus 100 may generate the optical panoramic image 102 in the arch shape according to the arch shape of the teeth. In addition, when the plurality of optical images 101 is a plurality of images taken by photographing at least one side surface selected from the lingual side and the labial side of the teeth, the imaging apparatus 100 may generate the optical panoramic image 102 in the linear shape so that the optical panoramic image 102 corresponds to a shape when the teeth are viewed from the lingual side and the and the labial side. In addition, when the optical panoramic image 102 is displayed in conjunction with the X-ray panoramic image 201, the imaging apparatus 100 may generate the optical panoramic image 102 in the linear shape so that a photographing direction of the optical panoramic image 102 corresponds to a photographing direction of the X-ray panoramic image 201. An additional configuration of this will be described with reference to FIG. 4A and FIG. 4B.

According to an embodiment of the present disclosure, the imaging apparatus 100 may acquire the X-ray panoramic image 201 taken from the X-ray radiography apparatus 300. At this time, the X-ray panoramic image 201 may be an image already taken for the intraoral cavity including the teeth arrangement of the subject person, and may be stored in the imaging apparatus 100. That is, the imaging apparatus 100 may be configured to search and retrieve the X-ray panoramic image 201 for the subject person, the X-ray panoramic image 201 being already taken.

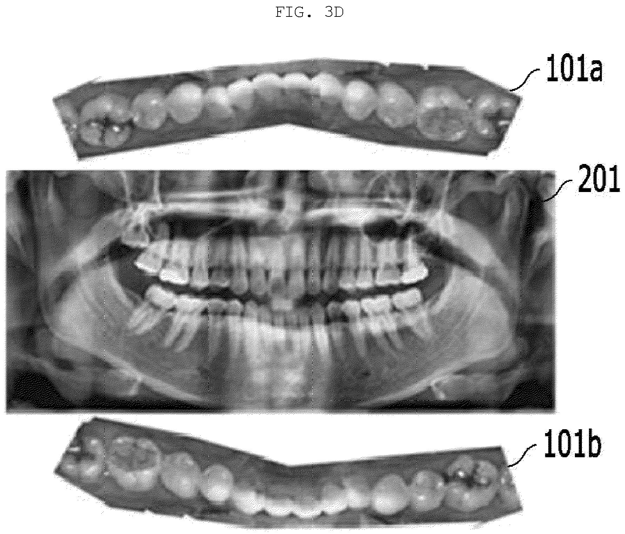

Referring to FIG. 3D, the imaging apparatus 100 may display the optical panoramic image 102 through the display device 400 in conjunction with the X-ray panoramic image 201. Specifically, the imaging apparatus 100 may display an optical panoramic image 101a of the upper teeth of the subject person on an upper side of the X-ray panoramic image 201 and may display an optical panoramic image 101b of the lower teeth of the subject person on a lower side of the X-ray panoramic image 201 so that the optical panoramic image 101a and the optical panoramic image 101b correspond to the positions of the upper teeth and the lower teeth of the subject person displayed in the X-ray panoramic image 201. However, the present disclosure is not limited thereto.

For example, the imaging apparatus 100 may be configured to display at least one tooth included in the optical panoramic image 102 and at least one tooth included in the X-ray panoramic image by matching the tooth included in the optical panoramic image and the tooth included in the X-ray panoramic image each other on the basis of the tooth number.

Specifically, the imaging apparatus 100 may identify the tooth number of each tooth for the X-ray panoramic image 201, may match each tooth displayed in the optical panoramic image 102 corresponding to the tooth number to each other, and may display any one tooth in the optical panoramic image 102 and any one tooth in the X-ray panoramic image 201 having the same identification number in conjunction with each other.

For example, when a selection input for any one tooth in the optical panoramic image 102 is received through the input part, the imaging apparatus 100 may display at least one tooth in the optical panoramic image 102 and the tooth matching with the corresponding tooth in the optical panoramic image 102 so that the tooth in the optical panoramic image 102 and the tooth in the X-ray panoramic image 201 are distinguished from other teeth. For another example, on the basis of any one tooth in the optical panoramic image 102 and the tooth in the X-ray panoramic image 201 matching with the corresponding tooth in the optical panoramic image 102, the size, the position, the angle and so on of the optical panoramic image 102 and the X-ray panoramic image 201 may be adjusted so that the two panoramic images are capable of being checked or compared with each other. However, the present disclosure is not limited thereto.

FIG. 4A and FIG. 4B are views illustrating an example of an X-ray panoramic image and an optical panoramic image according to an embodiment of the present disclosure.

Specifically, FIG. 4B may be a view illustrating the optical panoramic image 102 generated on the basis of the plurality of optical images 101 taken from any one side surface selected from the lingual side and the labial side of the teeth and formed so as to correspond to the teeth arrangement of the X-ray panoramic image 201 in the linear shape.

As described above, the imaging apparatus 100 may provide the X-ray panoramic image 201 and optical panoramic image 102 such that the X-ray panoramic image 201 and the optical panoramic image 102 correspond to each other. Furthermore, as illustrated in FIG. 4A and FIG. 4B, the imaging apparatus 100 may generate and provide the X-ray panoramic image 201 and the optical panoramic image 102 such that the X-ray panoramic image 201 and the optical panoramic image 102 are formed in shapes corresponding to each other on the basis of the same image.

In addition, as described above, the linear shape indicates an arrangement shape of the teeth photographed from any one side surface selected from the lingual side and the labial side of the teeth, but is not limited thereto. Furthermore, the imaging apparatus 100 may substantially generate the optical panoramic image 102 in an arrangement form corresponding to the teeth in the X-ray panoramic image 201. As an example, such an arrangement of the teeth may include a smile shape and a reverse smile shape.

In addition, the imaging apparatus 100 may display the optical panoramic image 102 on the X-ray panoramic image 201 such that the optical panoramic image 102 corresponds to a magnification rate (a magnification image) of the teeth in the X-ray panoramic image 201. Specifically, when an input for enlarging or reducing the size of the X-ray panoramic image 201 is received through the input part, the imaging apparatus 100 may display the optical panoramic image 102 so that the optical panoramic image 102 is enlarged or reduced so as to correspond to the magnification rate of the X-ray panoramic image 201.

Alternatively, the imaging apparatus 100 may display the optical panoramic image 102 in an arrangement shape corresponding to the teeth in the X-ray panoramic image 201, or may display the optical panoramic image 102 according to an arrangement shape of identified teeth or a magnification rate by identifying the tooth in the X-ray panoramic image 201 and identifying the arrangement shape of the identified teeth or the magnification rate in order to display the optical panoramic image 102 on the X-ray panoramic image 201 so that the optical panoramic image 102 corresponds to the magnification rate (the magnification image) of the teeth.

As described above, since the teeth and the teeth arrangement in the optical panoramic image 102 are compatible with and are capable of corresponding to the teeth and the teeth arrangement in the x-ray panoramic image 201, the accuracy and the convenience of analyzing the image may be maximized.

According to an embodiment of the present disclosure, when at least one lesion is detected in at least one image selected from the optical panoramic image 102 and the X-ray panoramic image 201, the imaging apparatus 100 may display at least one lesion detected in each of the optical panoramic image 102 and the X-ray panoramic image 201.

Specifically, the imaging apparatus 100 may detect the presence or absence of at least one lesion from each of the optical panoramic image 102 and the X-ray panoramic image 201, and may extract a region for the detected lesion from each of the optical panoramic image 102 and the X-ray panoramic image 201 when the lesion is present. In addition, the display device 400 may display the region for the lesion extracted from each of the optical panoramic image 102 and the X-ray panoramic image 201 by overlapping the region on the panoramic images.

At this time, the lesion detected from the optical panoramic image 102 may be at least one selected from a tooth crack, a tooth coloration, a tooth tartar, a tooth plaque, and a tooth caries, and the lesion detected from the X-ray panoramic image 201 may be at least one selected from an alveolar bone resorption, a supernumerary tooth, a congenitally missing tooth, a deciduous tooth (a primary tooth), and an eruption of a permanent tooth (a baby tooth), and a wisdom tooth (third molar). However, the present disclosure is not limited thereto.

For example, when a caries is detected in any one molar from the optical panoramic image 102, the imaging apparatus 100 may extract a region containing the caries present in the corresponding molar and may overlap and display the extracted region for the caries onto a corresponding molar region in the X-ray panoramic image 201, so that the lesion that was capable of being checked only through the optical panoramic image 102 is capable of being checked while the X-ray panoramic image 201 is observed.

For example, when an alveolar bone resorption is detected in any one tooth from the X-ray panoramic image 201, the imaging apparatus 100 may extract a region where the alveolar bone resorption is present in the corresponding tooth and may overlap and display the extracted region for the alveolar bone resorption onto a corresponding tooth region in the optical panoramic image 102, so that the lesion that was capable of being checked only through the X-ray panoramic image 201 is capable of being checked while the optical panoramic image 102 is observed.

Accordingly, lesions detected from each of the optical panoramic image 102 and the X-ray panoramic image 201 are capable of being detected from the panoramic images different from each other by using the imaging apparatus 100, so that the imaging apparatus 100 may help the medical personnel more effectively explain to the patient not only the position and type of the lesions but also another dental problem that may be caused by the lesions or other dental problems that may be a cause of the lesions.

According to an embodiment of the present disclosure, the imaging apparatus 100 may adjust the optical panoramic image 102 and the X-ray panoramic image 201 on the basis of a Computer Aided Design (CAD), and may provide CAD information on the basis of the adjustment.

As an example, the imaging apparatus 100 may identify and extract at least one feature from the optical panoramic image 102 and the X-ray panoramic image 201, and may adjust the optical panoramic image 102 and the X-ray panoramic image 201 with each other on the basis of the feature. At this time, a learning model previously trained may be used for identifying and extracting features.

For example, in order to increase the accuracy of CAD information, the imaging apparatus 100 may utilize the learning model trained to identify any one tooth that may be characterized from the optical panoramic image 102 and the X-ray panoramic image 201, or may utilize a database-based machine learning and so on in which learning is performed for the size, the position, the shape of various teeth on the basis of a plurality of panoramic images stored in a storage space such as a database (DB) and so on.

As described above, since the optical panoramic image for the entire teeth arrangement is generated and provided by stitching the plurality of individual images along the teeth arrangement direction, the present disclosure may support a comprehensive observation of a dental lesion and a comprehensive observation of a relationship between the tooth where the lesion is present and a state of surrounding teeth.

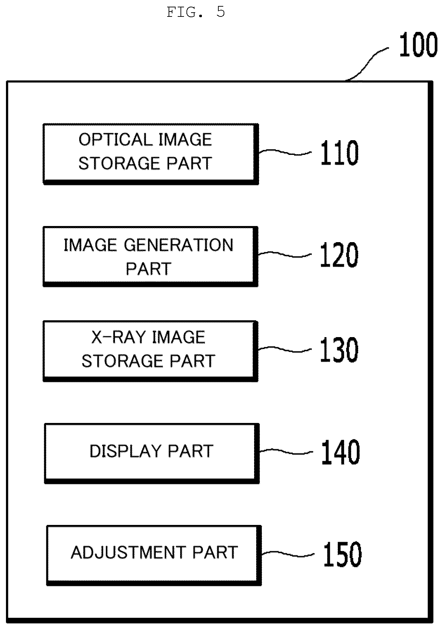

FIG. 5 is a block diagram schematically illustrating an intraoral imaging apparatus according to an embodiment of the present disclosure.

Referring to FIG. 5, the imaging apparatus 100 may include comprises an optical image storage part 110, an image generation part 120, an X-ray image storage part 130, and a display part 140, and an adjustment part 150.

According to an embodiment of the present disclosure, the optical image storage part 110 may store the plurality of optical images 101 taken from the intraoral photographing apparatus 200. Here, each of the plurality of optical images 101 may be an image in which at least one side surface selected from the occlusion surface, the lingual side, and the labial side of the teeth is photographed.

According to an embodiment of the present disclosure, the image generation part 120 may generate the optical panoramic image 102 for the entire teeth arrangement by stitching the plurality of optical images 101 along the teeth arrangement direction. Specifically, when the plurality of optical images 101 is taken, the image generation part 120 may generate the optical panoramic image 102 on the basis of the sensor value acquired from the inertial sensor included in the intraoral photographing apparatus 200 that is configured to capture the plurality of optical images 101.

According to an embodiment of the present disclosure, the X-ray image storage part 130 may store the X-ray panoramic image 201 for the intraoral cavity photographed by the X-ray radiography apparatus 300.

According to an embodiment of the present disclosure, the display part 140 may display the optical panoramic image 102 in conjunction with the X-ray panoramic image 201. Specifically, the display part 140 may display at least one tooth included in the optical panoramic image 102 and at least one tooth included in the X-ray panoramic image 201 by matching each tooth in the optical panoramic image 102 and the X-ray panoramic image 201 each other.

According to an embodiment of the present disclosure, the adjustment part 150 may adjust the optical panoramic image 102 and the X-ray panoramic image 201 on the basis of the CAD.

Hereinafter, an operation flow of the present disclosure will be briefly described on the basis of the detailed description described above.

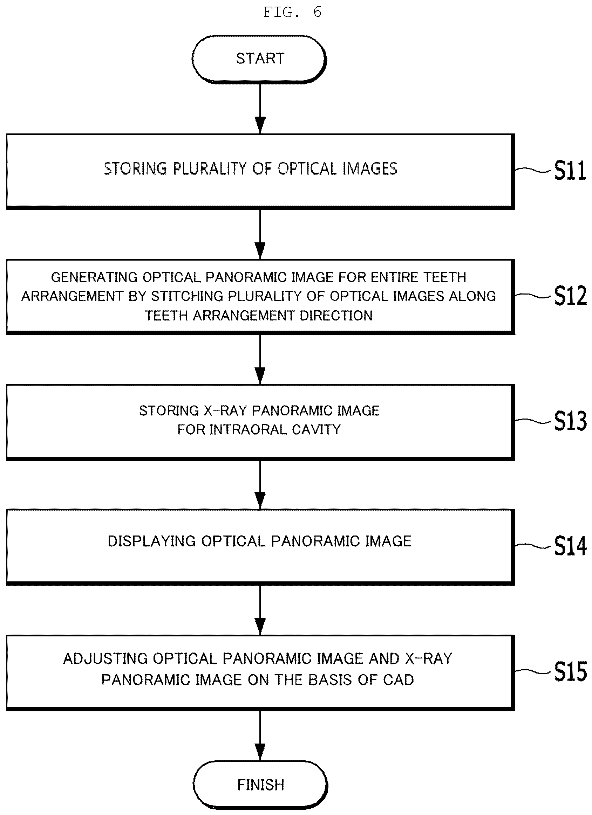

FIG. 6 is an operation flow chart illustrating an intraoral image displaying method according to an embodiment of the present disclosure.

The intraoral image displaying method illustrated in FIG. 6 may be performed by the intraoral imaging apparatus 100 described above. Therefore, even when a description is omitted hereinafter, the description of the intraoral imaging apparatus 100 may be equally applied to the description of the intraoral image displaying method.

Referring to FIG. 5, in a process S11, the optical image storage part 110 may store the plurality of optical images 101 taken by the intraoral photographing apparatus 200. Here, each of the plurality of optical images 101 may be an image acquired by using at least one light source selected from the white light source, the red light source, and the blue light source. In addition, each of the plurality of optical images 101 may be an image in which at least one side surface selected from the occlusion surface, the lingual side, and the labial side of the teeth is photographed.

Next, in a process S12, the image generation part 120 may generate the optical panoramic image 102 for the entire teeth arrangement by stitching the plurality of optical images 101 along the teeth arrangement direction. Specifically, in a process S12, the image generation part 120 may generate the optical panoramic image 102 on the basis of a tooth number for at least one tooth included in each of the plurality of optical images 101. At this time, the tooth number may be assigned on the basis of the sensor value acquired from the inertial sensor included in the intraoral photographing apparatus 200 configured to acquire the plurality of optical images 101, the sensor value being acquired when the plurality of optical images 101 is acquired.

Next, in a process S13, the X-ray image storage part 130 may store the X-ray panoramic image 201 acquired from the X-ray radiography apparatus 300.

Next, in a process S14, the display part 140 may display the optical panoramic image 102 in conjunction with the X-ray panoramic image 201. Specifically, in the process S14, the display part 140 may match and display at least one tooth included in the optical panoramic image 102 with at least one tooth included in the X-ray panoramic image 201 on the basis of the tooth number. In addition, in the process S14, when at least one lesion is detected in at least one image selected from the optical panoramic image 102 and the X-ray panoramic image 201, the display part 140 may display at least one lesion in each of the optical panoramic image 102 and the X-ray panoramic image 201.

Next, in a process S15, the adjustment part 150 may adjust the optical panoramic image 102 and the X-ray panoramic image 201 on the basis of the CAD.

In the description described above, the process S11 to the process S15 may be further divided into additional processes or may be combined into fewer processes according to an embodiment of the present disclosure. In addition, some processes may be omitted as required, and the order between the processes may be changed.

The intraoral image displaying method according to an embodiment of the present disclosure may be implemented as a program instruction executable by a variety of computer mechanisms and may be recorded on a computer-readable medium. The computer-readable medium may include a program instruction, a data file, a data structure, and so on alone or in combination. The program instruction recorded on the medium may be designed and configured specifically for an embodiment of the present disclosure or may be publicly known and available to those who are skilled in the field of computer software. Examples of the computer-readable recording medium include a magnetic medium, such as a hard disk, a floppy disk, and a magnetic tape, an optical medium, such as a compact disk read-only memory (CD-ROM), a digital versatile disc (DVD), and so on, a magneto-optical medium such as a floptical disk, and a hardware device specially configured to store and perform the program instruction, such as a read-only memory (ROM), a random access memory (RAM), a flash memory, and so on. Examples of the computer instruction include not only machine language code generated by a compiler, but also include high-level language code executable by a computer using an interpreter and so on. The hardware device may be configured to operate as one or more software modules in order to perform the operation of the present disclosure, and vice versa.

Furthermore, the intraoral image displaying method described above may be implemented in a form of a computer program or an application executed by the computer, the computer program or the application being stored in the recording medium.

The aforementioned description of the present disclosure is used for exemplification, and it can be understood by those skilled in the art that the present disclosure can be easily modified in other detailed forms without changing the technical spirit or requisite features of the present disclosure. Therefore, it should be appreciated that the aforementioned exemplary embodiments are illustrative in all aspects and are not restricted. For example, respective constituent elements described as single types can be distributed and implemented and, similarly, constituent elements described to be distributed can also be implemented in a coupled form.

The scope of the present disclosure is represented by claims to be described below rather than the detailed description, and it is to be interpreted that the meaning and scope of the claims and all the changes or modified forms derived from the equivalents thereof come within the scope of the present disclosure.

## Claims

1. An intraoral image displaying method comprising:
storing a plurality of optical images for the intraoral cavity including a teeth arrangement;
generating an optical panoramic image for the entire teeth arrangement by stitching the plurality of optical images along a teeth arrangement direction; and
displaying the optical panoramic image.

2. The intraoral image displaying method of claim 1, wherein, in the generating of the optical panoramic image, the optical panoramic image is generated on the basis of a sensor value acquired from an inertial sensor included in an intraoral imaging apparatus configured to acquire the plurality of optical images, the sensor value being acquired when the plurality of optical images is acquired.

3. The intraoral image displaying method of claim 1, further comprising storing an X-ray panoramic image of the intraoral cavity,
wherein, in the displaying of the optical panoramic image, at least one tooth included in the optical panoramic image and at least one tooth included in the X-ray panoramic image are matched with each other and are displayed.

4. The intraoral image displaying method of claim 1, wherein each of the plurality of optical images is an image in which at least one side surface of the occlusion surface, the lingual side, and the labial side of the teeth is photographed.

5. An intraoral imaging apparatus comprising:
an optical image storage part in which a plurality of optical images for the intraoral cavity including a teeth arrangement is stored;
an image generation part configured to generate an optical panoramic image for the teeth arrangement by stitching the plurality of optical images along a teeth arrangement direction; and
a display part configured to display the optical panoramic image.

6. The intraoral imaging apparatus of claim 5, further comprising an X-ray image storage part in which an X-ray panoramic image is stored,
wherein the image generation part is configured to match and display at least one tooth included in the optical panoramic image and at least one tooth included in the X-ray panoramic image.

7. An intraoral imaging system comprising:
an intraoral photographing apparatus configured to photograph a plurality of optical images for the intraoral cavity including a teeth arrangement; and
an intraoral imaging apparatus configured to generate an optical panoramic image for the entire teeth arrangement by stitching the plurality of optical images for the intraoral cavity along a teeth arrangement direction, the intraoral imaging apparatus being configured to display the optical panoramic image.

8. The intraoral imaging system of claim 7, further comprising an X-ray radiography apparatus configured to radiograph an X-ray panoramic image of the intraoral cavity,
wherein the intraoral imaging apparatus is configured to match and display at least one tooth included in the optical panoramic image and at least one tooth included in the X-ray panoramic image.
